(19) Europäisches Patentamt
European Patent Office
Office européen des brevets

(11) Veröffentlichungsnummer: **0 044 446**
**A2**

# (12) EUROPÄISCHE PATENTANMELDUNG

(21) Anmeldenummer: 81105050.9

(22) Anmeldetag: 30.06.81

(51) Int. Cl.³: **A 61 K 31/78**
A 61 K 31/79, A 61 K 31/785

(30) Priorität: 12.07.80 DE 3026574

(43) Veröffentlichungstag der Anmeldung:
27.01.82 Patentblatt 82/4

(84) Benannte Vertragsstaaten:
AT BE CH DE FR GB IT LI NL SE

(71) Anmelder: BAYER AG
Zentralbereich Patente, Marken und Lizenzen
D-5090 Leverkusen 1, Bayerwerk(DE)

(72) Erfinder: Bömer, Bruno, Dr.
Gustav-Freytag-Strasse 2
D-5090 Leverkusen 1(DE)

(72) Erfinder: Wolf, Gerhard Dieter, Dr.
Wilhelm-Busch-Strasse 29
D-4047 Dormagen 5(DE)

(72) Erfinder: Bartl, Herbert, Dr.
Eichendorffweg 10
D-5068 Odenthal(DE)

(72) Erfinder: Bierling, Robert, Dr.
Merlinweg 18 b
D-5600 Wuppertal 1(DE)

## (54) Antitumoral wirkende Mittel.

(57) Carbonsäureamidgruppenhaltige Polymere der allgemeinen Formeln I und II sind Antitumormittel mit großer therapeutischer Breite.

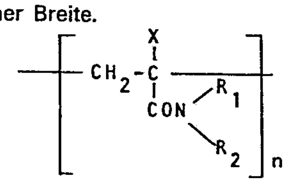

1

Darin bedeuten
X = H oder $C_1$-$C_4$-Alkyl,
besonders bevorzugt H und $CH_3$;
$R_1$ und $R_2$ können gleich oder verschieden sein und H eine Alkyl-, Cycloalkyl, Aryl- oder heterocyclische Gruppe mit 1 - 10 C-Atomen bedeuten, wobei die Summe der Kohlenstoffatome in den Resten $R_1$ und $R_2$ vorzugsweise ≤ 11 ist, sowie $R_1$ und $R_2$ eine Alkylengruppe mit 3 - 5 C-Atomen oder $-CH_2-CH_2-O-CH_2-CH_2-$ ist, und
n eine Zahl von 7 - 500 bedeutet,
und

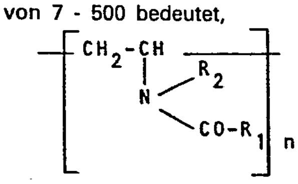

11

in welcher
$R_1$ und $R_2$ gleich oder verschieden sein können und H, eine Alkyl-, Cycloalkyl-, Aryl- oder heterocyclische Gruppe mit 1 - 10 C-Atomen, vorzugsweise H oder $C_1$ - $C_4$-Alkyl, sowie $R_1$ und $R_2$ eine Alkylengruppe mit 3 - 5 C-Atomen, und n eine Zahl von 7 - 500 bedeutet.

EP 0 044 446 A2

Croydon Printing Company Ltd.

0044446

BAYER AKTIENGESELLSCHAFT
Zentralbereich
Patente, Marken und Lizenzen

5090 Leverkusen-Bayerwerk
Si/ABc
II (Pha)

## Antitumoral wirkende Mittel

Die vorliegende Erfindung betrifft die Verwendung an sich bekannter, nicht ionischer, wasserlöslicher carbonsäureamidgruppenhaltiger Polymerisate als antitumoral wirkende Mittel.

Es ist schon bekannt geworden, daß copolymere, komplexe $Cu^{II}$- und $Co^{II}$-Salze der Ethylenmaleinsäure gegen das Walker-Sarkom wirksam sind [J. Med. Chem. 12 (1969), 1180].

Weiterhin wurden Polykationen verschiedenen Typs, z.B. Polyamidamine, Poly-N-morpholinoethylacrylamid und N-Oxid-Polymere auf Hemmung der Metastasierung geprüft mit dem Ergebnis, daß nur die Tumorzellaussaat, nicht aber das Metastasenwachstum in situ bzw. Lymphknotenmetastasen beeinflußt werden konnten [J. Med. Chem. 16 (1973), 496].

Ferner wurde die Wirksamkeit von Polymeren mit Carboxylgruppen gegen das Sarkom 180 in Abhängigkeit vom Molekulargewicht, der Ladungsdichte sowie der Metall-Bindungsfähigkeit der Carboxylgruppen beschrieben [Dissertation Abstr. Intern. B 33 (1973), 5745].

Polyanionen, z.B. Poly-ammoniumacrylat, Acrylsäure-acrylamid-Copolymere sowie Copolymere aus Ethylen- und Maleinsäureanhydrid sollen im Zusammenhang mit ihrer Antitumorwirkung einen Heparin-ähnlichen Effekt, daneben eine Virushemmung besitzen und außerdem die Immunreaktionen steigern [J. Med. Chem. 17 (1974), 1335].

Aus allen diesen Arbeiten geht hervor, daß die Antitumorwirkung der bisher untersuchten Polymeren an den verwendeten experimentellen Tumoren oft nur an der unteren Grenze der Signifikanz liegt und verschiedentlich nur auf prophylaktische oder adjuvantive Effekte beschränkt ist. Nachteilig erscheint außerdem, daß die zitierten Untersuchungen vielfach an allogenen, zur Spontanregression neigenden Mäusetumoren und nicht systematisch und unter kliniknahen Versuchsanordnungen durchgeführt wurden. Meist fehlen Angaben zur Toxizität der Präparate, obwohl die Applikation hoher Dosierungen von Substanzen mit einem Molekulargewicht von über 30 000 eine mangelhafte Ausscheidung bzw. Speicherung in den Geweben vermuten läßt.

Außerdem ist eine gewisse antitumorale Wirkung von Emulgatoren, die Polyethylenoxidketten eingebaut enthalten, bekannt geworden. So wurde Polyoxiethylen-sorbitan-monooleat (Tween 80) für die Immunisierung gegen den hyperdiploiden Ehrlich-Tumor verwendet [Experientia 29 (1973), 710].

Ein Blockcopolymerisat aus Poly-oxypropylen und Polyoxyethylen (Pluronic F 68) erwies sich als wirksam gegen

den Metastasenangang des Walker 256-Ascites-Tomors, wahrscheinlich durch Beeinflussung der Blutgerinnungsfähigkeit [Cancer 29 (1972), 171]. Diese Präparate sind bekanntermaßen hochwirksame Emulgatoren und sind aus diesem Grunde nicht sehr gut verträglich, insbesondere bei parenteraler Applikation.

Ebenfalls wurden antitumoral wirkende Mittel beschrieben, die durch einen Gehalt an mindestens einem wasserlöslichen Homo- oder Copolymerisat, das das 1,3-Dihydroxy-2-methylen-propan und/oder dessen Derivate enthält, charakterisiert sind [DOS 2 705 189]. Ähnliche Präparate mit ähnlicher Wirkung sind wasserlösliche Homo- oder Copolymerisate, die 3,4-Dihydroxibuten-1 oder Hydroxi-alkyl-(meth)-acrylate sowie deren Derivate oder auch Derivate des Allylalkohols polymerisiert oder copolymerisiert enthalten [DOS 2 740 082].

B.S. Michaels et. al [Nature <u>212</u> (1966), 101] beschrieben die virushemmende Wirkung von Polyvinylpyrrolidon (PVP). Bei in vitro-Versuchen hemmten PVP-Konzentrationen $> 10$ mg/ml bei Herpes simplex- und Vaccinia-Virus deutlich die Plaquebildung, während eine Konzentration von 1 mg/ml wirkungslos war.

Bei in vivo-Versuchen an 3 Tagen alten Küken [B.S Michaels et al., Proc. Amer. Ass. Cancer Res. <u>8</u>, Apr. (1967), 45] wurde bei simultaner Applikation von Rous-Sarkom-Virus (RSV) und PVP eine Inhibierung der virusbedingten Tumorbildung beobachtet. Eine Vorbehandlung der Küken bewirkte nur einen kleinen Effekt, während eine Gabe von PVP 6 h nach der Virusinfektion noch wirksam war.

Durch diese Ergebnisse wird in keiner Weise nahegelegt, daß PVP bereits in sehr geringen Dosen sowohl prophylaktisch (6 Tage vor der Tumortransplantation (TT)), als auch kurativ (2 Tage nach der TT) signifikante Hemmwirkungen an nicht virusinduzierten Tumoren besitzt.

W. Regelson et al. /Nature 186 (1960), 778-780/ untersuchten die Antitumorwirkung synthetischer Polyelektrolyte wie Polyacrylsäure, Polymethacrylsäure und hydrolysierten bzw. aminolysierten Ethylen-Maleinsäureanhydrid-Copolymerisaten. Sie fanden durch Wirkungsvergleich der Dicarbonsäure-, der Amid-Carbonsäure- und der Diamid-Form von Ethylen-Maleinsäureanhydrid-Copolymerisaten, daß für eine signifikante Tumorinhibierung mindestens eine ionisierbare Carboxylgruppe erforderlich ist. Versuche dieser Autoren mit Polyacrylamiden ergaben in hohen Dosen (800 mg/kg, MW 60-70000 und 400 mg/kg, MW 120000) eine negative bzw. eine nicht signifikante, sehr geringe positive Tumorhemmwirkung.

Es wurde nun überraschenderweise gefunden, daß auch nichtionische, wasserlösliche, carbonsäureamidgruppenhaltige Polymerisate an soliden Tumoren in einem breiten Dosisbereich von 0,5 - 500 mg/kg, vorzugsweise 2,5 bis 250 mg/kg, unter kliniknahen Versuchsanordnungen und Applikationsarten signifikante Tumorhemmwirkungen besitzen. Die akuten Toxizitäten der Substanzen sind gering ($LD_{50}$ i.v.: >2500 mg/kg), so daß die Substanzen eine ungewöhnlich große therapeutische Breite besitzen.

Unter nichtionischen Polymerisaten sind solche zu verstehen, die bei der Anwendung unter physiologischen Bedingungen nicht elektrolytisch dissoziieren, d.h. insbesondere keine Amino-,Carboxyl- oder Sulfonsäuregruppen aufweisen.

Die erfindungsgemäß zu verwendenden carbonsäureamidgruppenhaltigen Polymeren besitzen folgende allgemeinen Formeln I und II:

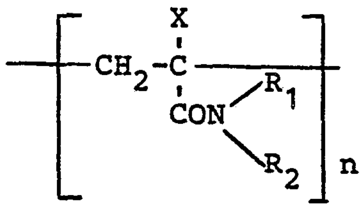

(I)

Darin bedeuten
X = H oder $C_1$-$C_4$-Alkyl,
besonders bevorzugt H und $CH_3$;

$R_1$ und $R_2$ können gleich oder verschieden sein und H, eine Alkyl-, Cycloalkyl, Aryl- oder heterocyclische Gruppe mit 1 - 10 C-Atomen bedeuten, wobei die Summe der Kohlenstoffatome in den Resten $R_1$ und $R_2$ vorzugsweise $\leq$ 11 ist, sowie
$R_1$ + $R_2$ eine Alkylengruppen mit 3 - 5 C-Atomen oder $-CH_2-CH_2-O-CH_2-CH_2-$ ist, und
n eine Zahl von 7 - 500 bedeutet

und

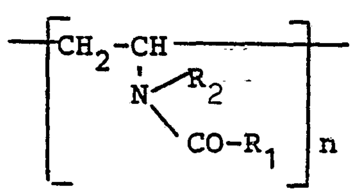

(II)

in welcher

$R_1$ und $R_2$ gleich oder verschieden sein können und H, eine Alkyl-, Cycloalkyl-, Aryl- oder heterocyclische Gruppe mit 1 - 10 C-Atomen, vorzugsweise H oder $C_1$-$C_4$-Alkyl, sowie
$R_1$ + $R_2$ eine Alkylengruppe mit 3-5 C-Atomen, und
n eine Zahl von 7 - 500 bedeutet.

Die Polymeren können, ausgehend von den entsprechenden Monomeren, nach bekannten Methoden, zum Beispiel durch radikalische Polymerisation, hergestellt werden. Die Polymerisation kann dabei in einem Lösungsmittel, welches Monomer und Polymer löst (Lösungspolymerisation), in einem Lösungsmittel, welches nur das Monomere löst (Fällungspolymerisation), oder auch ohne Lösungsmittel (Substanzpolymerisation) durchgeführt werden.

Dabei wählt man bevorzugt solche Polymerisationsverfahren, bei denen man Produkte mit einer relativ engen Molekulargewichtsverteilung erhält. Die nicht umgesetzten Monomeranteile sollten aus den Produkten entfernt werden, weil sie üblicherweise eine höhere Toxizität aufweisen, als die Polymeren.

Als Monomere zur Herstellung von Polymeren mit der allgemeinen Formel I seien namentlich genannt: Acrylamid, Methacrylamid, N-Methylacrylamid, N-Ethylacrylamid, N,N-Dimethylacrylamid, N-Isopropylacrylamid und N-Acryloylmorpholin.

Als Monomere zur Herstellung von Polymeren mit der allgemeinen Formel II seien genannt:

N-Vinylformamid, N-Vinylacetamid, N-Vinylpropionamid, N-Vinylbuttersäureamid, N-Vinyl-N-methylacetamid, N-Vinyl-N-ethylacetamid, N-Vinylpyrrolidon, N-Vinylpiperidon und N-Vinylcaprolactam.

Auch die Copolymerisation von zwei oder mehreren der vorgenannten Monomeren führt zu erfindungsgemäß zu verwendenden Polymeren. Durch Copolymerisation kann beispielsweise eine gewünschte bessere Wasserlöslichkeit der Polymeren erreicht werden.

Außerdem können, falls gewünscht, auch bis zu 70 Gew.-%, bezogen auf die Gesamtmenge der Monomeren, anderer nicht ionischer Monomerer, wie z.B. Acrylnitril, Ester der Acrylsäure oder Methacrylsäure mit gesättigten Monoalkoholen mit 1 - 8 C-Atomen, Vinylester aliphatischer Carbonsäuren mit 1 - 4 C-Atomen, Vinylether mit Alkylresten mit 1 - 8 C-Atomen, Vinylhalogenide, wie Vinylchlorid und Vinylidenchlorid, sowie Diester von Maleinsäure und Fumarsäure mit gesättigten aliphatischen Monoalkoholen zusammen mit einem oder mehreren der carbonsäureamidgruppenhaltigen Monomeren copolymerisiert werden, wobei die Menge des Comonomeren dadurch begrenzt ist, daß ein in physiologischer Kochsalzlösung bei 20-40°C zu mindestens 0,5 Gew.-%igen Lösungen lösliches Polymer entsteht.

Vorzugsweise wird die Polymerisation durch radikalbildende Initiatoren, wie aliphatische Azoverbindungen, Diacylperoxide, oder Percarbonsäureester bzw. Perkohlensäureester ausgelöst. Das Molekulargewicht der entstehenden Polymeren kann dabei durch verschiedene bekannte Maßnahmen beeinflußt werden. So erhält man bei Verwendung steigender Initiatormengen Polymerisate mit abnehmendem Molekulargewicht. Eine weitere Möglichkeit der Verringerung des Molekulargewichtes besteht im Zusatz von molekulargewichtsregelnden Stoffen, wie z.B. Mercaptanen oder in der Verwendung bestimmter Lösungsmittel, wie z.B. Isopropanol.

Die erfindungsgemäß zu verwendenden carbonsäureamidgruppenhaltigen Polymerisate besitzen mittlere Molekulargewichte $\bar{M}_n$ von etwa 300 bis etwa 50 000. Polymere mit Molekulargewichten unterhalb etwa 25 000 werden bevorzugt, da bei diesen mit einer Ausscheidung durch die Nieren gerechnet werden kann.

Die erfindungsgemäßen antitumoralen Mittel werden hergestellt, indem man die carbonsäureamidgruppenhaltigen Polymeren in physiologischer Kochsalzlösung löst. Sie können aber auch in Tabletten, Kapseln, Säfte und andere für die orale Applikation übliche Zubereitungen eingearbeitet werden. Die dabei erhaltenen Zubereitungen werden intraperitoneal, intravenös, intramuskulär oder oral appliziert. Der therapeutische Dosisbereich reicht von etwa 0,5 - 500 mg/kg, vorzugsweise 5 - 250 mg/kg.

Die erfindungsgemäßen Mittel haben bei äußerst niedriger Toxizität eine stark antitumorale Wirkung gegen tierische und menschliche Tumoren und sollen daher zur Bekämpfung von durch Tumoren verursachten Erkrankungen verwendet werden.

Die erfindungsgemäßen Mittel wurden in folgender Weise am Carcinom EO 771 auf C 57 BL/6 - Mäusen getestet:

Tierart: C 57 BL/6 - Mäuse, Inzucht (SPF)

Verfahren: Stammhaltung: Am 14. - 20. Tag nach letzter Transplantation subcutane Verimpfung einer Zellsuspension des Carcinoms EO 771 in 0,5 ml Phosphat-gepufferter 0,9 %iger NaCl-Lösung auf C 57 BL/6 - Mäuse.

Vorbereitung von Screening-Testen: Gleiches Verfahren wie bei Stammhaltung des Tumors, jedoch subcutane Verimpfung einer Suspension von 5 x $10^4$ Tumorzellen in 0,5 ml Phosphat-gepufferter 0,9 %iger NaCl-Lösung.

Behandlung: Einmalige, intramuskuläre Injektion der geforderten Präparatlösungen am 6. Tag vor bzw. am 2. Tag nach der Tumortransplantation.

Versuchsdauer: 18 - 22 Tage nach der Tumortransplantation. Dann Abtötung der Tiere, Präparation und Wägung der subcutanen Tumoren.

Auswertungsparameter: Hemmung des Tumorwachstums durch Bestimmung des durchschnittlichen Tumorgewichts von Kontrollen und behandelten Tiergruppen sowie Errechnung

des Tumorgewichts- (TG-) - Index nach der Formel:

$$\text{TG-Index} = \frac{\text{Ø Tumorgewicht der behandelten Tiergruppen}}{\text{Ø Tumorgewicht der Kontrollgruppe}}$$

<u>Beurteilung der Testergebnisse:</u> TG-Index 0,8 - 0,6 = Wirkungsandeutung; 0,6 - 0,4 = deutliche Wirkung: <0,4 = gute Wirkung.

Tabelle 1: Prüfergebnisse am Carcinom EO 771 bei jeweils einmaliger i.m.-Applikation

| Polymeres von Beispiel-Nr. | Dosis mg/kg | Behandlungstag + | Tumorgewichts-Index |
|---|---|---|---|
| 1 | 2,5 | - 6 | 0,21 |
| | 2,5 | + 2 | 0,28 |
| 2 | 2,5 | - 6 | 0,22 |
| | 250 | + 2 | 0,55 |
| 3 | 10 | - 6 | 0,32 |
| | 10 | + 2 | 0,28 |
| 4 | 250 | - 6 | 0,33 |
| | 250 | + 2 | 0,53 |
| 5 | 10 | - 6 | 0,51 |
| | 50 | + 2 | 0,68 |
| 6 | 60 | - 6 | 0,53 |
| | 50 | + 2 | 0,51 |
| 7 | 10 | - 6 | 0,26 |
| | 2,5 | + 2 | 0,40 |

+Behandlungstag: - 6 = 6 Tage vor Tumortransplantation
+ 2 = 2 Tage nach Tumortransplantation

Die Tumorgewichts-Indices der in der Tabelle 1 aufgeführten Präparate lassen erkennen, daß die Substanzen an dem gut standardisierbaren Leittestsystem Carcinom EO 771 der Maus bei verschiedenen Dosierungen sowie an verschiedenen Behandlungstagen (prophylaktisch sowie kurativ) signifikante Tumorhemmwirkungen zu induzieren vermögen.

Beispiel 1

400 ml destillierter Isopropylalkohol werden vorgelegt, die Apparatur wird 3 x auf 100 mbar evakuiert und mit Stickstoff gefüllt und das vorgelegte Lösungsmittel zum Sieden erhitzt. 100 g Acrylamid und 1 g Dilauroylperoxid werden in 600 ml Isopropanol gelöst. Die Monomerlösung wird vom Sauerstoff befreit und unter Überleiten von Stickstoff innerhalb 3 Stunden zur gerührten siedenden Vorlage getropft. Es wird 3 Stunden unter Rückfluß nachgerührt. Das Polymerisatpulver wird abfiltriert, 2 x mit je 1 l Aceton ausgekocht, erneut abfiltriert und bei 60°C unter vermindertem Druck bis zur Gewichtskonstanz getrocknet. Man erhält 95 g Polymerpulver. $[\eta]$ = 0,064 (bestimmt bei 25°C in 0,9 %iger wäßriger NaCl-Lösung).

Beispiel 2

400 ml destilliertes Ethylacetat werden vorgelegt. Die Apparatur wird 3 x auf 100 mbar evakuiert und mit Stickstoff gefüllt und das vorgelegte Lösungsmittel zum Sieden erhitzt.

80 g Methacrylamid und 10 g Azoisobuttersäurediethylester werden in 800 ml destilliertem Ethylacetat gelöst. Die Monomerlösung wird vom Sauerstoff befreit und unter Überleiten von Stickstoff innerhalb 3 Stunden zur gerührten siedenden Vorlage getropft. Es wird 3 Stunden unter Rückfluß nachgerührt. Das Polymerisat wird abgesaugt, 2 x mit je 1 l Aceton ausgekocht, erneut abfiltriert und bei 60°C im Vakuum getrocknet.

Ausbeute: 66 g Polymeres
[η] = 0,082 (bestimmt bei 25°C in 0,9 %iger wäßriger NaCl-Lösung).

Beispiel 3

1400 ml destillierter Isopropylalkohol werden vorgelegt, wie in Beispiel 2 beschrieben, vom Sauerstoff befreit und zum Sieden erhitzt. 70 g Acrylamid, 30 g frisch destilliertes N-Vinylpyrrolidon und 1 g Dilauroylperoxid werden in 600 ml destilliertem Isopropylalkohol gelöst. Die Monomerlösung wird vom Sauerstoff befreit und unter Überleiten von Stickstoff innerhalb 3 Stunden gleichmäßig zur gerührten siedenden Vorlage getropft. Anschließend wird 3 Stunden unter Rückfluß nachgerührt, das Polymerisat abgesaugt, mehrfach intensiv mit Aceton gewaschen und bei 60°C im Vakuum getrocknet.

Ausbeute: 84 g
[η] = 0,042 (bestimmt bei 25°C in 0,9 %iger wäßriger NaCl-Lösung).

Beispiel 4

Analog zu Beispiel 3 wird eine Lösung von 50 g Acrylamid, 50 g N-Vinylpyrrolidon und 1 g Lauroylperoxid in 600 ml Isopropylalkohol polymerisiert.

Ausbeute: 79 g
[η] = 0,049 (bestimmt bei 25°C in 0,9 %iger wäßriger NaCl-Lösung).

Beispiel 5

50 g N-Vinylacetamid und 5 g Azoisobuttersäuredinitril werden in 300 ml Isobutylalkohol gelöst. Die Lösung wird sorgfältig vom Sauerstoff befreit und unter Stickstoff 3 Stunden zum Sieden erhitzt. Die Polymerlösung wird dann im Vakuum auf etwa 200 ml eingeengt und in 2,5 l Aceton eingerührt. Das ausgefällte Polymerisat wird intensiv mit Aceton gewaschen und im Vakuum getrocknet.

Ausbeute: 27 g
$[\eta]$ = 0,12 (bestimmt bei 25°C in 0,9 %iger wäßriger NaCl-Lösung).

Beispiel 6

500 ml n-Heptan werden vorgelegt, wie in Beispiel 1 beschrieben, vom Sauerstoff befreit und unter Rühren auf 95°C erhitzt. Innerhalb 2,5 Stunden wird eine sauerstofffreie Mischung von 95 g N-Vinyl-N-methyl-acetamid, 5 g Azoisobuttersäurediethylester und 150 ml n-Heptan gleichmäßig unter Sauerstoffausschluß zugetropft. Das Reaktionsgemisch wird 2 Stunden bei 95°C nachgerührt, auf Raumtemperatur gekühlt und das ausgefallene Polymerisat abgesaugt, mit n-Heptan gründlich gewaschen und getrocknet. Ausbeute: 85,3 g
$[\eta]$ = 0,21 (bestimmt bei 25°C in 0,9 %iger wäßriger NaCl-Lösung)

Beispiel 7

200 ml destillierter Isobutylalkohol werden vorgelegt. Die Apparatur wird 3 x auf 100 mbar evakuiert und mit Stickstoff gefüllt und das vorgelegte Lösungsmittel zum Sieden erhitzt. 300 g frisch destilliertes N-Vinylpyrrolidon und 7,5 g Azoisobuttersäurediethylester werden mit Isobutanol auf 1 200 ml aufgefüllt. Die Monomerlösung wird vom Sauerstoff befreit und unter Überleiten von Stickstoff mit 400 ml/h gleichmäßig zur gerührten siedenden Vorlage getropft. Anschließend wird 2 Stunden unter Rückfluß nachgerührt. Der Ansatz wird dann mit 3 l n-Butylacetat versetzt, und es werden bei 300 - 400 mbar 3 l Lösungsmittelgemisch abdestilliert. Nach dem Abkühlen auf Raumtemperatur wird das überstehende Lösungsmittel dekantiert. Das Polymerisat wird noch 2 x mit je 2 l Butylacetat 2 Stunden bei 120°C extrahiert und das Extraktionsmittel nach dem Abkühlen dekantiert. Dann wird das Polymerisat bei 120°C am Rotationsverdampfer im Vakuum von Lösungsmittelresten befreit und mechanisch zerkleinert.

Ausbeute: 173 g Polymerisat
Molekulargewicht $\bar{M}_n$ = 3 400 (bestimmt durch Dampfdruckosmometrie in DMF).

/$\bar{\eta}$7 = 0,051 (bestimmt bei 25°C in 0,9 %iger wässriger NaCl-Lösung).

Patentansprüche

1. Antitumoral wirkende Mittel, gekennzeichnet durch einen Gehalt an mindestens einem nicht ionischen, wasserlöslichen Carbonsäureamidgruppen enthaltenden Vinyl- bzw. Acrylpolymeren.

2. Antitumoral wirkende Mittel nach Anspruch 1, dadurch gekennzeichnet, daß das wasserlösliche Polymere aus einem oder mehreren Monomeren der allgemeinen Formel Ia

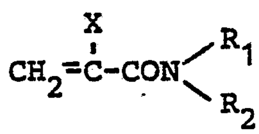

(Ia)

in welcher

X = H oder $C_1$-$C_4$-Alkyl bedeutet,
$R_1$ und $R_2$ gleich oder verschieden sein können und H, eine Alkyl-, Cycloalkyl-, Aryl- oder heterocyclische Gruppe mit 1-10 C-Atomen, sowie $R_1$ + $R_2$ eine Alkylengruppe mit 3 - 5 C-Atomen oder $-CH_2-CH_2-O-CH_2-CH_2-$ bedeuten,

aufgebaut ist.

3. Antitumoral wirkende Mittel nach Anspruch 1, dadurch gekennzeichnet, daß das wasserlösliche Polymere aus einem oder mehreren Monomeren der allgemeinen Formel IIa

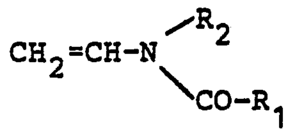

(IIa)

in welcher

$R_1$ und $R_2$ gleich oder verschieden sein können und H, eine Alkyl-, Cycloalkyl-, Aryl- oder heterocyclische Gruppe mit 1-10 C-Atomen, sowie $R_1$+$R_2$ eine Alkylengruppe mit 3-5 C-Atomen bedeuten, aufgebaut ist.

4. Antitumoral wirkende Mittel nach Anspruch 1, dadurch gekennzeichnet, daß sie Copolymerisate aus Monomeren der Formeln Ia und IIa enthalten.

5. Antitumoral wirkende Mittel nach Anspruch 1, dadurch gekennzeichnet, daß sie wasserlösliche Copolymerisate aus
0-99 Mol % Monomeren der Formel Ia,
0-99 Mol % Monomeren der Formel IIa und
1-70 Mol % anderen nichtionischen Vinyl- oder Acrylmonomeren enthalten.

6. Verfahren zur Herstellung eines antitumoral wirkenden Mittels, dadurch gekennzeichnet, daß man mindestens ein nicht ionisches, wasserlösliches Carbonsäureamidgruppen enthaltendes Vinyl- oder Acrylpolymer nach den Ansprüchen 1 - 5 in einer physiologischen Kochsalzlösung löst, oder in Zubereitungen für die orale Applikation einarbeitet.

7. Verfahren zur Behandlung von Tumoren, dadurch gekennzeichnet, daß man dem Organismus von an Tumoren

erkrankten Menschen antitumoral wirkende Mittel gemäß den Ansprüchen 1-5 systemisch oder lokal appliziert.